# EUROPEAN PATENT SPECIFICATION

(11) **EP 1 507 568 B1**
(45) Date of publication and mention of the grant of the patent: **28.03.2007**
(21) Application number: 03735471.9
(22) Date of filing: 27.05.2003
(51) Int. Cl.: A61M 16/16

(54) **Device for heating and moistening a breathing gas**
Vorrichtung zum Anwärmen und Befeuchten eines Beatmungsgerät
Dispositif de conditionnement de gaz médical

(30) Priority: 29.05.2002 EP 02447099
(43) Date of publication of application: 23.02.2005
(73) Proprietor: Anthony, Jean-Michel, B-1840 Londerzeel (BE)
(72) Inventor: Anthony, Jean-Michel, B-1840 Londerzeel (BE)
(74) Representative: Brants, Johan P.E.
(86) International application number: PCT/EP2003/005557
(87) International publication number: WO 2003/099367

(56) References cited:
- EP-A- 0 567 158
- EP-A- 1 138 341
- BE-A- 1 003 559
- US-A- 5 769 071
- US-A- 6 095 505

## Description

### Field of the invention

The present invention relates to devices for the conditioning of medical gas and more in particular to humidifiers.

### Background

Under physiological conditions, the nose functions to provide active warming and humidifying of breathing air. However, when a patient is undergoing artificial respiration the nose is shunted by a flexible tube, whose distal end is inserted into the trachea. In order to prevent unwanted effects such as cough stimulus or draining of the mucous membranes, the breathing air supplied must be treated. This is necessary, because the nose and throat mucous membranes, which normally take over this task, are no longer available under these circumstances. The moistening and warming treatment of the supplied breathing gases, which is essential for normal function of the lungs, is nowadays performed by devices usually composed of a humidifying and heating apparatus.

Humidifiers are known in the art. Humidifiers are used to warm and moisten medical gas supplied to a patient. Humidifiers are available in which the water for humidifying the circulating gas is held in a heated reservoir and the circulating gas which is to be humidified is passed over said reservoir. These prior art humidifiers generally comprise a source of air (or other mixture of gases), a humidification chamber including a source of water and a heating means to vaporise the water, and a conduit to convey the humidified gases to the patient or user. These humidifiers may further comprise evaporation means, usually made of blotting paper.

U.S. Patent No. 3,638,926 discloses humidification and heating apparatus which comprises a spirally wound resistance-heated plate in thermal contact with a sheet of water absorbent blotting paper. The air from a respirator travels along a spiral passage formed by the plate, the water surface at a reservoir, and a lid to produce a substantially saturated vapor at a desired temperature.

Generally the reservoir sized to accommodate enough water to avoid frequent refilling, the entire volume of water in the reservoir is heated to and maintained at the desired temperature so that establishing maintaining and changing the desired water temperature are more difficult than for lesser volumes of water. Furthermore, the water in these devices stagnates and can be prone to bacterial proliferation. The maintenance and cleaning of these humidifiers is furthermore difficult to perform. In addition, their complex design may not allow their reutilization after a single use.

WO 97/07845 discloses an apparatus for compensating heat and humidity loss in a device that administers or restitutes warm and humid air to a patient. The device described in this document is however not suited for the supply of high volume of humid and warm medical gas which is essential for patient in need of high airflow.

EP-A-567 158 discloses a humidifier according to the preamble of claim 1.

It is a main object of the present invention to provide a device for the conditioning of medical gas, which can maintain the heat level and the humidity proportion of said medical gas at the requested level. It is another object of the present invention to provide a device for the conditioning of medical gas wherein said air is provided with a high flow and moisture output. It is a further object to provide a device for the conditioning of medical gas which is an all-in-one device having heating and humidifying functions, which can be connected to a gas supply duct at an arbitrary localization so that a very great flexibility of the device is assured. A further object of the invention is to provide a device for the conditioning of medical gas, which can be easily sterilized. It is yet another object of the present invention to provide a device for the conditioning of medical gas which can be reusable. It is yet a further object of the present invention to provide a device for the humidification of gas supplied to a patient wherein said gas is a breathing gas. It is yet a further object of the present invention to provide a device for the humidification of gas supplied to a patient wherein said gas is used for the purpose of laparoscopic surgery.

### Summary of the invention

The present invention relates to a device for conditioning medical gas comprising a housing provided with an inlet for non treated medical gas and an outlet for the treated medical gas, wherein in said housing conditioning means are provided for the humidification and warming of said medical gas, comprising a rigid evaporating body, a water supply inlet and a heating device, wherein said water supply inlet is connected to the housing such that a thin layer of water is able to be inserted between the heating device and the rigid evaporating body. In an embodiment of the present invention, said conditioning means are arranged between said inlet and said outlet.

More in particular the present invention, as claimed, relates to a device for conditioning medical gas characterized in that said rigid evaporating body and said heating device are arranged in a concentric way forming a space between them and wherein said water supply inlet is connected to the housing such that a thin layer of water is able to be inserted in said space between the heating device and the rigid evaporating body. Said rigid evaporating body and said heating device can be of any suitable shape so as to allow a space when arranged concentrically. Said rigid evaporating body and heating device may be of cylindrical shape, such as to form a cylindrical space when arranged concentrically. Other suitable shapes include but are not limited to rectangular, octagonal, and the like. In a preferred embodiment, said space between the heating device and the rigid evaporating body is cylindrical.

The device according to the invention permits the humidification and warming of high volumes and flow of medical gas supplied to an individual in need thereof. The water supply inlet is connected to the housing such that a thin layer of water is able to be inserted in the space between the heating device and the rigid evaporating body. In a preferred embodiment the space between the heating device and the rigid evaporating body may be ranging from 0.1 to 0.001 cm.

The space between the heating device and the rigid evaporating body may also be larger than 0.1 cm, and may be in such case further provided therein with a water absorbing product such as blotting paper, sponge, ceramic, synthetic water absorbing material and the like. For example, the space between the heating device and the rigid evaporating body may be provided with a second rigid evaporating body with water absorbing properties. The thin layer of water would be inserted in said second water absorbing properties.

In another embodiment, of the present invention, the device according to the invention may be provided with the heating device in close contact with the rigid evaporating body, said heating device or rigid evaporating body being provided with cylindrical or longitudinal grooves thereby allowing a thin layer of water is able to be inserted in said grooves.

As used herein a thin layer of water relates to a layer ranging from 0.1 to 0.001 cm. The water evaporation in thin layer permits to avoid pressure build-up in the space between the rigid evaporating body and the heating device. This water evaporation in thin layer in the space allows water feeding by gravity for example and precise vapor output by regulation of the temperature of the heating element.

In the present invention, said rigid evaporating body is micro-porous. As used herein rigid evaporating body also refers to monolithic body. Furthermore, said rigid evaporating body has substantial water impermeable and Water vapor permeable characteristics. Suitable rigid evaporating body can be made of any rigid material known in the art with water impermeable and water vapor permeable characteristics. Said rigid evaporating body is preferably made of solid material having meshes with water impermeable and water vapor permeable characteristics. In an embodiment, the rigid evaporating body can be made of material having a mesh size ranging from 0.05 µm to 1 µm, preferably having a mesh size of 0.2 µm. The use of a rigid evaporating body in the present device provides several advantages such as accuracy and stability of the device. The rigid body does not deform due to heat and pressure caused by the water when heated. Furthermore, the space between said rigid evaporating body and the heating element is kept constant due to said rigidity, and further offers a security against waterleaks.

In the present invention, said rigid evaporating body is made of ceramic material such as porous alumina treated or not treated with a hydrophobic product such as water repellent product. In a preferred embodiment, said rigid evaporating body is a micro-porous ceramic cylinder, which is preferably hollow. In order to obtain water impermeable and water vapor permeable characteristics for said ceramic cylinder, the pores of said cylinder may be impregnated with a hydrophobic product. In said rigid evaporating body can be made of a monolith of micro-porous PTFE. Said micro-porous PTFE monolith is preferably made of a hollow cylinder, with a wall thickness ranging from 1 to 10, preferably from 3 to 10 mm, and most preferably from 5 to 6 mm. The rigid evaporating body made of ceramic or of monolithic PTFE has the advantage of being robust, heat resistant and can be further easily sterilized, using means known in the art such as oven sterilization for example.

Said device has the advantage of being heat resistant and may be cleaned, replaced and/or sterilized easily. Said device can therefore be reusable.

In another embodiment of the present invention, the device according to the invention can be provided with a further insulating casing surrounding the housing of said device. This casing permits the thermal insulation of the device.

In another embodiment, the housing walls of the device according to the invention can be additionally surrounded by a second heating device in a concentric way. This allows the reduction of water condensation and provides further warming of said medical gas. According to this embodiment, the additional heating device may be further enclosed in an insulating casing in order to thermally insulate said device.

The device has a housing wherein conditioning means are provided for the humidification and warming of medical gas comprising a rigid evaporating body, a water inlet and a heating device characterized in that said rigid evaporating body and said heating device are arranged in a concentric way forming a space between them. In an embodiment, said rigid evaporating body is surrounding said heating device. In another embodiment, said heating device is surrounding said rigid evaporating body.

The device of the present invention ensures that the correct humidity of the circulating gas is always maintained, whilst warming said gas simultaneously. Furthermore, the device of the present invention provides the advantage of being space-saving and easy to handle. Indeed most prior-art devices are composed of a humidifying apparatus and a warming apparatus in the form of a water reservoir and a heating means to vaporise the water, and optionally provided with evaporation means. The assembly of such parts is cumbersome, and provides with disadvantages when such apparatuses need to be replaced or cleaned. Moreover, mistakes may be made when connecting said assembly to a gas supply and to a patient, as too many inlet, outlet and tubing are involved. The present invention provides with a humidifying and heating unit all combined in one device, wherein medical gas is humidified and warmed simultaneously. Said device has the advantage of having at least one gas inlet to be connected to the gas supply and one gas outlet to connect to a patient. As there is only one way to connect the new device, there can be no confusion. Furthermore, the device according to the invention has the advantage of having a thin layer of water circulating and being continuously evaporated, whereas in prior-art devices the water to be evaporated is stagnating at 37°C to 40°C thereby favouring the development of microorganism.

The device of the present invention permits to condition said breathing with a high flow and moisture output, which is particularly suited in case of difficult artificial respiration situations, such as for instance in artificial respiration of premature babies with high respiration rates and small vital capacities.

The present invention will be further disclosed in detail hereunder wherein preferred embodiments of the device of the present invention are disclosed in details. The description is only given by way of example and does not limit the invention. The reference numbers relate to the hereto-annexed figures.

### Brief description of the figures

Figure 1 represents a top-view of a preferred embodiment of a device according to the invention.
Figure 2 represents a cross-sectional view along the line II-II of the device of figure 1.
Figure 3 represents a cross-sectional view of another preferred embodiment of a device according to the invention.
Figure 4 represents a cross-sectional view of yet another preferred embodiment of a device according to the invention.

### Detailed description

The present invention relates to a device suitable for the conditioning of medical gas.

As used herein the term "medical gas" relates to any breathable gas suitable for an individual in need thereof. It also relates to any gas to be delivered to body cavities or organs of an individual in need thereof, such as for example such as normal air to be delivered via nose or mouth to the lungs, or O₂/CO₂ gas mixtures to be provided during laparoscopic surgery. As used herein the term "individual" relates to animals, preferably mammals, and more preferably human.

Said device is particularly suited for the conditioning of breathing air to be supplied to the lungs, and also for the conditioning of gas to be supplied to a body cavity such as in laparoscopic surgery.

As used herein the term "conditioning of medical gas" and/or "treatment of medical gas" refers to the humidification/moisturizing and the warming of said gas.

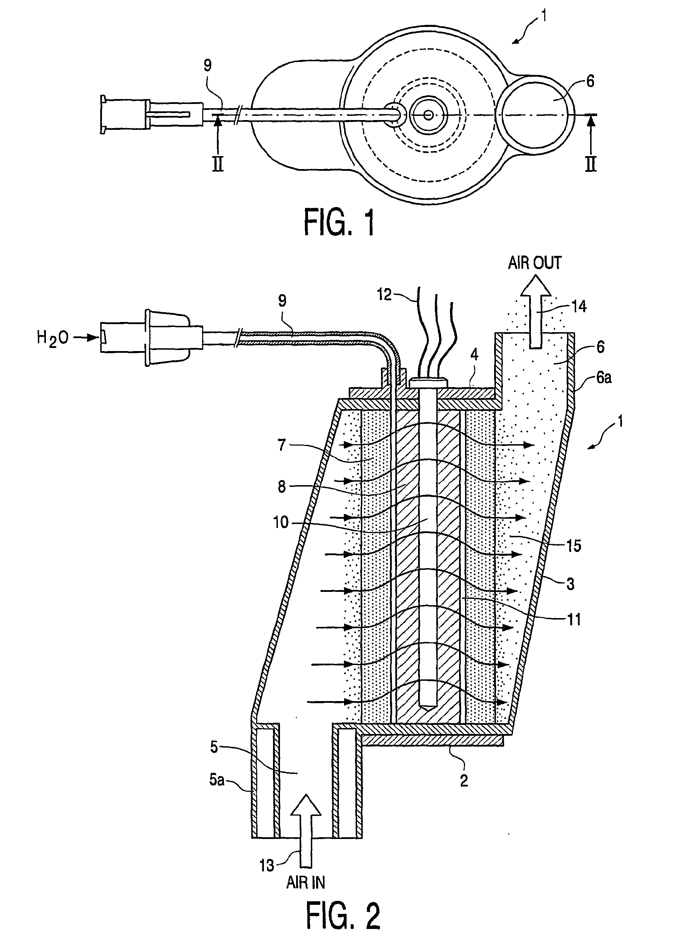

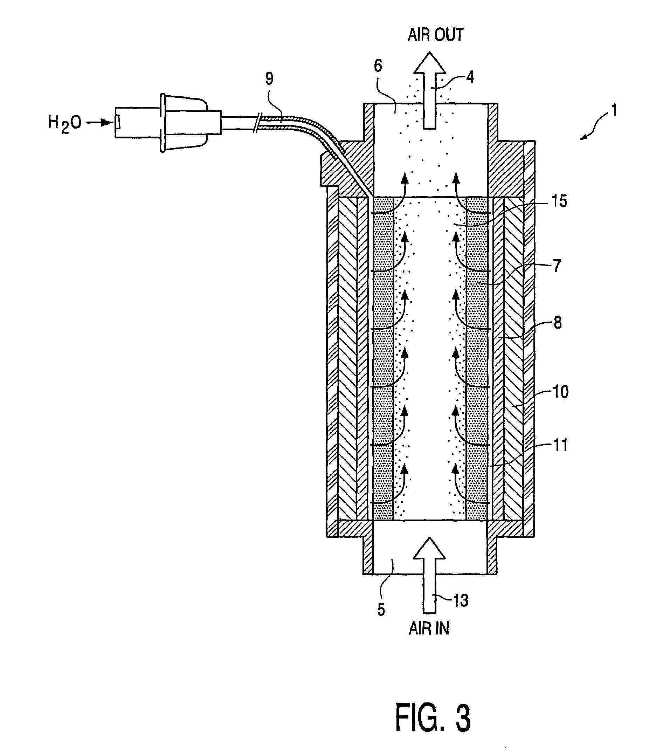

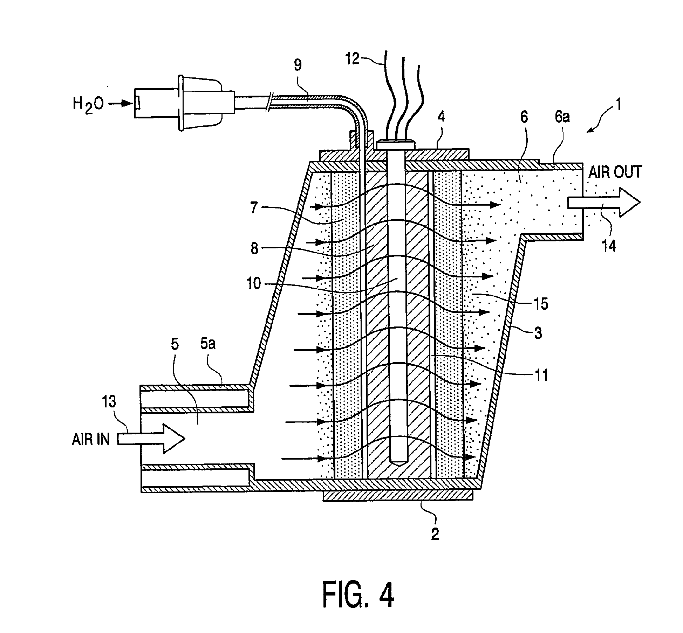

A device according to the invention is composed of a housing 1, which can be cylindrical as illustrated on figure 3, but can also have other suitable shapes. In a preferred embodiment, said device has a shape as illustrated in figures 2 or 4. The housing can be made of material suitable for medical uses. It is preferably made of plastic or metal.

As shown figure 2, said device may comprise a housing 1 having a bottom 2, a peripheral wall 3 and a cover 4, wherein said housing 1 is provided with an inlet opening 5 for the supply of medical gas as illustrated with arrow 13, and an outlet opening 6 from which the treated medical gas exits 14. Said housing 1 further comprises conditioning means for the humidification and warming of said gas, consisting essentially of a rigid evaporating body 7 arranged in said housing 1 between said inlet 5 and outlet 6, a heating device 8, and a water inlet 9. Said rigid evaporating body 7 and said heating device 8 are arranged in a concentric way forming a space 11 between them and wherein said water inlet 9 is connected to the housing such that a thin layer of water is able to be inserted in said space 11 between the heating device 8 and the rigid evaporating body 7. In said embodiment, the evaporating body 7 is surrounding the heating device 8. The action of the heating device 8 on the water supplied in the space 11 allows the production of water vapor schematically illustrated herein by dots 15. The passing gas thereby efficiently loads itself with water vapor 15. Said heating device 8 can be a self-adjusting resistance of the PTC-type. Said heating resistance 8 can be composed of a central heating component 10, which may be cylindrical and can be surrounded by a metallic element, as shown figure 2. Suitable metallic elements comprises but is not limited to any metallic elements conducting heat, such as aluminum, titanium, stainless steel, silver and the like. In a preferred embodiment anodized aluminum is used. In another embodiment, the housing 1 may be further surrounded by an additional heating device in a concentric way.

In another embodiment shown on figure 3, said device comprises a housing 1 having a bottom 2, and a cover 4, wherein said housing 1 is provided with an inlet opening 5 for the supply of medical gas as illustrated with arrow 13, and an outlet opening 6 for the treated medical gas as illustrated with arrow 14, further comprising conditioning means for the humidification and warming of said gas. Said conditioning means consist essentially of a rigid evaporating body 7 arranged in said housing 1 between said inlet 5 and outlet 6, a heating device 8, and a water inlet 9, characterized in that said rigid evaporating body 7 and said heating device 8 are arranged in a concentric way forming a space 11 between them and wherein said water inlet 9 is connected to the housing such that a thin layer of water is able to be inserted between the heating device 8 and the rigid evaporating body 7. As illustrated figure 3, said rigid evaporating body 7 is surrounded by said heating device 8 in a concentric way forming a space 11 between them. Moreover, the outlet 6 is located on the top of said rigid evaporating body 7 and the inlet 5 is located on the bottom of said rigid evaporating body 7. As show figure 3, said rigid evaporating body 7 is preferably a hollow cylinder. The action of the heating device 8 on the water supplied in the space 11 allows the production of water vapor schematically illustrated herein by dots 15. Said heating device 8 can be a self-adjusting resistance of the PTC-type. It can comprise a heating component 10, which may be cylindrical as shown figure 3, which surround a metallic element cylinder, such as an anodized aluminum cylinder. Although not shown herein the heating component 10 may have temperature sensors attached to it. Said temperature sensors 12 can be attached on the top or on the bottom of said heating component 10. In a preferred embodiment said temperature sensors are attached on the bottom of said heating component 10.

For the purpose of thermal insulation of the device and in order to permit the easy handling of the device as illustrated on figure 3, insulating walls are further provided in the housing between the cover 4 and the bottom 2, thereby surrounding the heating device 8.

Suitable rigid evaporating body 7 is preferably micro-porous and has substantial water impermeable and water vapor permeable characteristics. Suitable rigid evaporating body 7 can be made of any solid material known in the art with water impermeable and water vapor permeable characteristics. Said rigid evaporating body 7 is preferably made of solid material having meshes with water impermeable and water vapor permeable characteristics. In an embodiment, the rigid evaporating body 7 can be made of material having a mesh size ranging from 0.05 µm to 1 µm, preferably having a mesh size of 0.2 µm. The evaporating body 7 allows by its heat transfer properties the warming of the gas passing through it.

In the present invention, said rigid evaporating body 7 is made of ceramic material. In a preferred embodiment, said rigid evaporating body 7 is a porous ceramic cylinder. In order to obtain water impermeable and water vapor permeable characteristics for said ceramic cylinder, the pores of said cylinder are preferably impregnated with a hydrophobic product. The use of the rigid evaporating body 7 which pores are impregnated with a hydrophobic product allows only water vapor 15 to enter the device and the entrainment of water droplets by the gas stream passing through said device is avoided. Hydrophobic products for impregnating said evaporating body are known in the art. Examples of such products are silane, silica and products used in the art to render walls impermeable. In the present invention, said rigid evaporating body 7 is made of a monolith of micro-porous PTFE. Said micro-porous PTFE monolith is preferably made of a hollow cylinder, with a wall thickness ranging from 1 to 10 mm, preferably from 3 to 10 mm, most preferably from 5 to 6 mm. The hydrophobic nature of the PTFE allows the liquid water to be repelled from the pores, whereas water vapor 15 can pass through.

In a preferred embodiment, as shown figure 2, the device according to the invention may have openings in said housing 1 wherein said inlet 5 is located in the bottom 2 and said outlet 6 is located in the cover 4 of said housing 1.

In another preferred embodiment as shown figure 4, the device according to the invention may have openings in said housing 1, wherein said inlet 5 is located at the bottom of said peripheral wall 3 and said outlet 6 is located at the top of said peripheral wall 3.

The device as illustrated in figure 2 or 4 ensures a rapid and optimum conditioning of the gas flowing through the device owing to the large surface area of the rigid evaporating body 7. The housing of the device as presently designed optimizes the warming and humidification of the gas flowing through said device. Indeed, the partial obstruction of the gas flow by the conditioning means comprised in the device of figures 2 or 4, ensures a longer contact time with the conditioning means thereby permitting said gas to efficiently loads itself with water vapor 15 produced by the conditioning means.

According to an embodiment, the inlet 5 and outlet 6 have connection pipes, illustrated respectively as 5a and 6a on figures 2 and 4, planned for attaching a tube. Said inlet and outlet 5 and 6, and the corresponding connection pipes may be of identical or different sizes. The flow rate through the device according to the invention may be modulated by adjusting the position and the size of the inlet and outlet and/or of the corresponding connection pipes. If a reduced flow out of the device is needed the size of the outlet 6 can be reduced. The position of the inlet and outlet may also be adjusted so as to obtain the best conditioning of the air circulating through the device. The connecting pipe on the inlet can be connected to a gas supply tube. By gas supply tube must be understood the part of the tube that is connected to a gas supply, or anesthesia or a non illustrated respiratory apparatus, or gas supply for laparoscopic surgery. In a preferred embodiment as illustrated in figures 2 and 4, said connecting pipe 5a can be composed of two cylinders of different diameters which permit the adaptation of tubing of different diameters according to the application. The connecting pipe 6a on the outlet can be connected to tube, which supplies gas to a patient. Said connecting pipes may be cylindrical in a preferred embodiment. The connecting pipes in a further embodiment may be additionally heated by means known in the art to ensure the warming of the supplied medical gas to a desired temperature and consequently avoid water condensation.

The device according to the invention is characterized in that said device has in said housing 1, rigid evaporating body 7 and a heating device 8 arranged in a concentric way in said housing 1 between said inlet 5 and outlet 6.

In an embodiment of the present invention, said rigid evaporating body 7 in said housing 1, surround the heating device 8 in a concentric way forming a space 11 between them as shown figure 2.

In another embodiment of the present invention as illustrated on figure 3, said rigid evaporating body 7 is surrounded by said heating device 8 in a concentric way forming a space 11 between them. In this embodiment, the device according to the invention comprises a housing 1 provided with an outlet 6 located on the top of said rigid evaporating body 7 and an inlet 5 located on the bottom of said rigid evaporating body 7.

A water inlet 9 is connected to the housing such that a thin layer of water is able to be inserted in said space 11 between the heating device 8 and the rigid evaporating body 7. The thin layer of water may be a layer ranging from 0.1 to 0.001 cm. Example of thin layer of water in said device may be ranging from 0.08 to 0.01 cm. The water can flow through said water inlet 9 into said space 11 by simple capillarity, active pumping, diffusion and/or by gravity. In a preferred embodiment said water flows in by gravity. The water inlet 9 is connected to said space 11. The water to be supplied is preferably held in a water reservoir that is connected to said inlet 9. Said water reaches the interior of the space 11 via the water supply inlet 9. In order to optimize the effect of the gravity for the supply of water in the device according to the invention, the height of the water reservoir can be ranging from 70 cm to 120 cm. In an embodiment of the present invention as shown herein, said water inlet 9 is connected to said space 11 on the top of the device. In another preferred embodiment not shown herein, said water inlet 9 is connected on the bottom of the device according to the invention.

To achieve the maintenance at the desired level of heat and humidity of the gas that must be administered to a patient, the gas flows through the device according to the invention, entering through the inlet 5 passing through said conditioning means. Said gas loads itself with water vapor 15 produced by the action of the heating device 8 on the supplied water.

The heating device allows for temperatures in the range of 120 °C to 150 °C to be obtained. Said heating device 8 can be a self-adjusting resistance of the PTC-type. It can be composed of a central component 10, which may be cylindrical and can be surrounded by a metallic element as shown figure 2, or as illustrated figure 3, said heating device 8 comprises a metallic element cylinder surrounded by a central component 10. Suitable metallic elements comprises but is not limited to any metallic elements conducting heat, such as aluminum, titanium, stainless steel, silver and the like. In a preferred embodiment anodized aluminum is used. Furthermore, said component 10 may have temperature sensors 12 attached to it. Said temperature sensors 12 can be attached on the top of the device, as illustrated in figure 2, or can be attached on the component 10 on the bottom of said component 10. Said heating component 10 is preferably connected in any convenient manner to an electrical source of energy through power cables. Said component 10, may be provide with means for the plugging of said power cables and for the optional plugging of LED (Light Emitting Diode) indicator.

In order to maintain the largest possible quantity of humid gas brought at the desired temperature at a constant level and to supply it in this state to a patient, said rigid evaporating body 7 surround the heating device 8 (Figures 2, 4), or as shown figure 3, said heating device 8 surround said rigid evaporating body 7. Condensation can then be reduced or prevented by the help of the heating device, ensuring the circulation gas is maintained at the correct temperature not only by the water vapor in the device but also by the help of the evaporating body. The moisture 15 output can be established in a continuous manner from 10 to 50 ml/h by adjusting the temperature of the heating device 8. Owing to the large surface area of the rigid evaporating body, rapid and optimum conditioning of the passing through medical gas is ensured. Furthermore, due to the high working temperatures ranging from 120 °C to 150 °C in the device, a constant sterilization of the water vapor can be obtained, thereby preventing microbial contamination and infections.

A check valve may be mounted on the water inlet 9. Said check valve will help minimizing the effect of the counter-pressure due to the rigid evaporating body 7 which act as a vapor room, and also permit to reduce the effect of the water reservoir height difference.

In another embodiment according to the invention said housing 1 may have flanges surrounding the top and the bottom of the peripheral wall 3. The presence of said flanges allows the device to be seized particularly easily and surely. This provides for the easy handling of the device according to the invention. Said device can be securely seized from the side, so that slipping is practically impossible and disconnecting of said device becomes easy to perform and problem free. The housing of said device can also be provided with an insulating casing for thermal isolation of the device so that burning incidents are practically impossible.

The heat exchange processed by the heating device 8 can be bi-directional and can thus influence by its feedback characteristics the self-adjusting function of the heating resistance present in component 10, so that in case of a greater gas flow this resistance will evaporate a greater quantity of water. The resistances of the heating device according to the invention can be of the P.T.C. type.

The present device according to the invention is made in such a way that the cleaning and/or replacement of the device is easily performed, allowing the device to be reused. The present device is also suitable for a single use and can be also partially or totally disposable. All the components of said device are heat resistant and said device can therefore be sterilized and reusable. Moreover, the design and the assembly of said device is such that the device is compact and robust. This provides that during use of said device for humidifying and warming medical gas, there is not only an optimum conditioning of said gas for the patient, but furthermore protection against infection and saving in costs. Indeed microbial contamination is avoided in said device, by the means of high working temperature ranging from 120 °C to 150 °C and by the fact that the water is continuously evaporated. The device according to the invention may further have heating and humidifying bodies that may be detachable and may be readily cleaned and sterilized. The device may be of any size suitable to efficiently condition a medical gas depending on its utilization. Non-limiting example of suitable sizes for the device according to the invention may be ranging from 5 to 20 cm, preferably from 10 to 15, most preferably from 11 to 13 cm for the height of the device. The width of the device may be ranging from 6 to 3 cm, more preferably from 4 to 5 cm.

The present device according to the invention allows the conditioning of all the gas flowing through said device, as the gas is totally and simultaneously humidified and warmed when circulating through the conditioning means of said device.

The present invention provides therefore a device allowing the conditioning of medical gas, namely humidification and heating of said gas. It is easy to handle, connect and disconnect. Moreover, by strictly maintaining the temperature of the humid gas in the device under control, the formation of too much condensation water herein can be prevented.

Said device clearly help in humidifying high volume of supplied gas while maintaining at the desired heat level the water vapor in the tubes that administer or restitute gas to a patient and assuring a humidity level whereby a minimum of condensation occurs in these tubes. Additional water traps may be used on the expiration line to prevent accumulation of water in the tubing.

When used for conditioning breathing air, said device can be placed on the inspiration line of a breathing apparatus, and can be located either near the patient or near the air supply apparatus.

Two or more devices according to the invention can be used in series thereby ensuring the correct conditioning of high airflow for example. The device can also be combined with any existing classical humidifiers to increase their performances. Said device can also be used in combination with a heat and moisture exchanger (HME). Said device can be further coupled or integrated to an HME. When used in combination with an HME, or when couple or integrated with said HME said device may be of smaller dimension. Non-limiting example of suitable sizes for said smaller device may be ranging from 1 to 3 cm in height and 1 to 3 cm in width. It may be further connected with an artificial nose. Said device may be further used in all applications requiring the humidification and heating of gas. Said device according to the invention is thereby suitable for the conditioning of medical gas. It can also be applicable to anesthesia and all other medical procedures in which the delivery of gas to a body cavities or organs is required. For example, it can be used in laparoscopic surgery for the humidification and heating of the gas supplied in the peritoneal cavity. The benefit of this particular use may be to provide a systemic effect of local anesthesia to the peritoneal cavity during surgery and hence much reduced post-procedural pain.

Moreover, the device according to the invention can be connected to a breathing apparatus that administer or restitute gas to a patient. It may be placed anywhere in the breathing circuit, even close to the patient.

The present disclosure further relates to a method for the conditioning of medical gas, comprising the step of connecting a device according to the invention, to a gas supply and running the gas through said device.

The present disclosure further relates to a method for the conditioning of medical gas, comprising the step of connecting a device according to the invention, to a gas supply and running the gas through said device. The present invention also relates to a method for the conditioning of medical gas, wherein said gas is humidified and warmed, comprising the steps of supplying through an inlet opening non-treated medical gas, humidifying and warming said supplied gas through the action of evaporated water forced through a porous rigid body by heating up a small column of water.

## Claims

1. Device for conditioning medical gas comprising:
a housing (1) provided with an inlet (5) for non treated medical gas and an outlet (6) for the treated medical gas, wherein in said housing (1) conditioning means are provided for the humidification and warming of said medical gas, comprising an evaporating body (7), a water supply inlet (9) and a heating device (8), wherein said evaporating body (7) and said heating device (8) are arranged in a concentric way forming a space (11) between them and wherein said water supply inlet (9) is connected to said space (11) between the heating device (8) and the evaporating body (8),
**characterized in that** said evaporating body (7) is a micro porous rigid monolith made of ceramic, or made of PTFE having a wall thickness ranging from 1 to 10 mm.

2. Device according to claim 1, wherein said space (11) between the heating device and the rigid evaporating body (7) is cylindrical.

3. Device according to any of claims 1 to 2, wherein said rigid evaporating body (7) has substantial water impermeable and water vapor permeable characteristics.

4. Device according to any of claims 1 to 3, wherein said rigid evaporating body (7) has a mesh size ranging from 0.05 µm to 1 µm.

5. Device according to claim 4, wherein the mesh size of said rigid evaporating body (7) is 0.2 µm.

6. Device according to any of claims 1 to 5, wherein said conditioning means are arranged between said inlet (5) and said outlet (6).

7. Device according to any of claims 1 to 6, wherein said evaporating body (7) is surrounding said heating device (8).

8. Device according to any of claims 1 to 7, wherein said housing (1) is further surrounded by an additional heating device in a concentric way.

9. Device according to any of claims 1 to 6, wherein said heating device (8) is surrounding said evaporating body (7).

10. Device according to any of claims 1 to 9, **characterized in that** said housing (1) has a bottom (2), a peripheral wall (3) and a cover (4).

11. Device according to claim 10, wherein the inlet (5) is located in the bottom (2) and the outlet (6) is located in the cover (4) of said housing (1).

12. Device according to claim 10, wherein the inlet (5) is located at the bottom (2) of said peripheral wall (3) and the outlet (6) is located at the top of said peripheral wall (3).

13. Device according to any of claims 1 to 12, wherein said heating device (8) comprises a central heating component (10) which is surrounded by a metallic element.

14. Device according to any of claims 1 to 6, **characterized in that** said housing has a bottom (2), and a cover (4).

15. Device according to claim 14, wherein the outlet (5) is located on the top of the evaporating body (7) and the inlet (6) is located on the bottom of said evaporating body (7).

16. Device according to any of claims 14 or 15, wherein the evaporating body (7) is surrounded by the heating device (8) in a concentric way forming a space (11) between them.

17. Device according to any of claims 14 to 16, wherein said heating device (8) comprises a heating component (10) surrounding a metallic element cylinder.

18. Device according to any of claims 1 to 17, wherein the water inlet (9) is connected to the space (11) between the heating device (8) and the evaporating body (7).

19. Device according to any of claims 1 to 18, wherein said water inlet (9) comprises a check valve.

20. Device according to any of claims 1 to 19, wherein said water inlet (9) is connected to a water reservoir.

21. Device according to claim 20, wherein said water reservoir is at a height ranging from 70 to 120 cm.

22. Device according to any of claims 1 to 21, wherein said heating device (8) comprises temperature sensors (12) attached to it.

23. Device according to any of claims 1 to 22, wherein said inlet (5) and outlet (6) have connection pipe (5a, 6a) and planned for attaching tubes.

24. Device according to any of claims 1 to 23, wherein said housing (1) has flanges surrounding the top and the bottom of the peripheral wall (3).

## Patentansprüche

1. Vorrichtung für die Konditionierung medizinischen Gases, wobei die Vorrichtung das Folgende umfasst:
ein Gehäuse (1), das mit einem Einlass (5) für nicht behandeltes medizinisches Gas und einem Auslass (6) für das behandelte medizinische Gas versehen ist, wobei in dem Gehäuse (1) Konditionierungsmittel für die Befeuchtung und Erwärmung des medizinischen Gases bereitgestellt sind, wobei die Mittel einen Verdampfungskörper (7), einen Einlass für die Wasserversorgung (9) und eine Heizvorrichtung (8) umfassen, wobei der Verdampfungskörper (7) und die Heizvorrichtung (8) auf eine konzentrische Weise angeordnet sind, so dass ein Raum (11) zwischen ihnen ausgebildet wird, und wobei der Einlass für die Wasserversorgung (9) mit dem Raum (11) zwischen der Heizvorrichtung (8) und dem Verdampfungskörper (7) verbunden ist,
**dadurch gekennzeichnet dass** der Verdampfungskörper (7) ein mikroporöser starrer Monolith ist, der aus Keramik besteht, oder der aus PTFE besteht, das eine Wanddicke im Bereich von 1 bis 10 mm besitzt.

2. Vorrichtung gemäß Anspruch 1, wobei der Raum (11) zwischen der Heizvorrichtung und dem starren Verdampfungskörper (7) zylindrisch ist.

3. Vorrichtung gemäß einem der Ansprüche 1 oder 2, wobei der starre Verdampfungskörper (7) die Eigenschaften besitzt, im Wesentlichen wasserundurchlässig und wasserdampfdurchlässig zu sein.

4. Vorrichtung gemäß einem der Ansprüche 1 bis 3, wobei der starre Verdampfungskörper (7) eine Maschenweite im Bereich von 0,05 µm bis 1 µm besitzt.

5. Vorrichtung gemäß Anspruch 4, wobei die Maschenweite des starren Verdampfungskörpers (7) 0,2 µm beträgt.

6. Vorrichtung gemäß einem der Ansprüche 1 bis 5, wobei die Konditionierungsmittel zwischen dem Einlass (5) und dem Auslass (6) angeordnet sind.

7. Vorrichtung gemäß einem der Ansprüche 1 bis 6, wobei der Verdampfungskörper (7) die Heizvorrichtung (8) umringt.

8. Vorrichtung gemäß einem der Ansprüche 1 bis 7, wobei das Gehäuse (1) weiter auf eine konzentrische Weise von einer zusätzlichen Heizvorrichtung umringt ist.

9. Vorrichtung gemäß einem der Ansprüche 1 bis 6, wobei die Heizvorrichtung (8) den Verdampfungskörper (7) umringt.

10. Vorrichtung gemäß einem der Ansprüche 1 bis 9, **dadurch gekennzeichnet dass** das Gehäuse (1) einen Boden (2), eine periphere Wand (3) und eine Abdeckung (4) besitzt.

11. Vorrichtung gemäß Anspruch 10, wobei sich der Einlass (5) im Boden (2) befindet und sich der Auslass (6) in der Abdeckung (4) des Gehäuses (1) befindet.

12. Vorrichtung gemäß Anspruch 10, wobei sich der Einlass (5) am Boden (2) der peripheren Wand (3) befindet und sich der Auslass (6) am Kopfende der peripheren Wand (3) befindet.

13. Vorrichtung gemäß einem der Ansprüche 1 bis 12, wobei die Heizvorrichtung (8) einen zentralen Heizungsbestandteil (10) umfasst, der von einem Metallelement umringt wird.

14. Vorrichtung gemäß einem der Ansprüche 1 bis 6, **dadurch gekennzeichnet dass** das Gehäuse einen Boden (2) und eine Abdeckung (4) besitzt.

15. Vorrichtung gemäß Anspruch 14, wobei sich der Auslass (6) am Kopfende des Verdampfungskörpers (7) befindet und sich der Einlass (5) auf dem Boden des Verdampfungskörpers (7) befindet.

16. Vorrichtung gemäß einem der Ansprüche 14 oder 15, wobei der Verdampfungskörper (7) von der Heizvorrichtung (8) auf eine konzentrische Weise umringt wird, so dass ein Raum (11) zwischen ihnen ausgebildet wird.

17. Vorrichtung gemäß einem der Ansprüche 14 bis 16, wobei die Heizvorrichtung (8) einen Heizungsbestandteil (10) umfasst, der einen Metallelement-Zylinder umringt.

18. Vorrichtung gemäß einem der Ansprüche 1 bis 17, wobei der Wassereinlass (9) mit dem Raum (11) zwischen der Heizvorrichtung (8) und dem Verdampfungskörper (7) verbunden ist.

19. Vorrichtung gemäß einem der Ansprüche 1 bis 18, wobei der Wassereinlass (9) ein Kontrollventil umfasst.

20. Vorrichtung gemäß einem der Ansprüche 1 bis 19, wobei der Wassereinlass (9) mit einem Wasserreservoir verbunden ist.

21. Vorrichtung gemäß Anspruch 20, wobei sich das Wasserreservoir in einer Höhe im Bereich von 70 bis 120 cm befindet.

22. Vorrichtung gemäß einem der Ansprüche 1 bis 21, wobei die Heizvorrichtung (8) an sie angebrachte Temperatursensoren (12) umfasst.

23. Vorrichtung gemäß einem der Ansprüche 1 bis 22, wobei der Einlass (5) und der Auslass (6) Anschlussrohre (5 a, 6 a) besitzen, die für die Anbringung von Röhren ausgelegt sind.

24. Vorrichtung gemäß einem der Ansprüche 1 bis 23, wobei das Gehäuse (1) Flanschen besitzt, die das Kopfende und den Boden der peripheren Wand (3) umringen.

## Revendications

1. Dispositif de conditionnement de gaz médical comprenant :
un boîtier (1) ayant une entrée (5) pour du gaz médical non traité et une sortie (6) pour le gaz médical traité;, dans lequel un moyen de conditionnement pour l'humidification et le réchauffement dudit gaz médical est présent dans ledit boitier (1), comprenant un corps d'évaporation (7), une entrée d'alimentation d'eau (9) et un dispositif de chauffage (8), dans lequel ledit corps d'évaporation (7) et ledit dispositif de chauffage (8) sont disposés de façon concentrique formant entre eux un espace (11) et dans lequel ladite entrée d'alimentation d'eau (9) est connectée au dit espace (11) entre le dispositif de chauffage (8) et le corps d'évaporation (7),
**caractérisé en ce que** ledit corps d'évaporation (7) est un monolithe micro poreux rigide en céramique, ou en PTFE ayant une épaisseur de paroi allant 1 à 10 mm.

2. Dispositif selon la revendication 1, dans lequel ledit espace (11) entre le dispositif de chauffage et le corps d'évaporation rigide (7) est cylindrique.

3. Dispositif selon l'une quelconque des revendications 1 ou 2 ; dans lequel ledit corps d'évaporation rigide (7) a des caractéristiques substantielles d'imperméabilité à l'eau et de perméabilité à la vapeur d'eau.

4. Dispositif selon l'une quelconque des revendications 1 à 3, dans lequel ledit corps d'évaporation rigide (7) a une ouverture de maille allant de 0,05 µm à 1 µm.

5. Dispositif selon la revendication 4, dans lequel l'ouverture de maille du corps d'évaporation rigide (7) est de 0,2 µm.

6. Dispositif selon l'une quelconque des revendications 1 à 5, dans lequel ledit moyen de conditionnement est disposé entre ladite entrée (5) et ladite sortie (6) .

7. Dispositif selon l'une quelconque des revendications 1 à 6, dans lequel ledit corps d'évaporation (7) entoure ledit dispositif de chauffage (8).

8. Dispositif selon l'une quelconque des revendications 1 à 7, dans lequel ledit boitier (1) est en outre entouré de façon concentrique par un dispositif de chauffage additionnel.

9. Dispositif selon l'une quelconque des revendications 1 à 6, dans lequel ledit dispositif de chauffage (8) entoure ledit corps d'évaporation (7).

10. Dispositif selon l'une quelconque des revendications 1 à 9, **caractérisé en ce que** ledit boitier (1) a un fond (2), une paroi périphérique (3) et un couvercle (4).

11. Dispositif selon la revendication 10, dans lequel l'entrée (5) est localisée dans le fond (2) et la sortie (6) est localisée dans le couvercle (4) dudit boitier (1).

12. Dispositif selon la revendication 10, dans lequel l'entrée (5) est localisée à la base de ladite paroi périphérique (3) et la sortie (6) est localisée en haut de ladite paroi périphérique (3).

13. Dispositif selon l'une quelconque des revendications 1 à 12, dans lequel ledit dispositif de chauffage (8) comprend un composant central de chauffage (10) qui est entouré par un élément métallique.

14. Dispositif selon l'une quelconque des revendications 1 à 6, **caractérisé en ce que** ledit boitier a un fond (2) et un couvercle (4).

15. Dispositif selon la revendication 14, dans lequel la sortie (6) est localisée sur le dessus du corps d'évaporation (7) et l'entrée (5) est localisée en dessous dudit corps d'évaporation (7).

16. Dispositif selon l'une quelconque des revendications 14 ou 15, dans lequel le corps d'évaporation (7) est entouré par le dispositif de chauffage (8) de façon concentrique formant entre eux un espace (11).

17. Dispositif selon l'une quelconque des revendications 14 à 16, dans lequel ledit dispositif de chauffage (8) comprend un composant de chauffage (10) entourant un élément cylindre métallique.

18. Dispositif selon l'une quelconque des revendications 1 à 17, dans lequel l'entrée pour l'eau (9) est connectée à l'espace (11) entre le dispositif de chauffage (8) et le corps d'évaporation (7).

19. Dispositif selon l'une quelconque des revendications 1 à 18, dans lequel ladite entrée pour l'eau (9) comprend un clapet antiretour.

20. Dispositif selon l'une quelconque des revendications 1 à 19, dans lequel ladite entrée pour l'eau (9) est connectée à un réservoir d'eau.

21. Dispositif selon la revendication 20, dans lequel ledit réservoir d'eau est à une hauteur allant de 70 à 120 cm.

22. Dispositif selon l'une quelconque des revendications 1 à 21, dans lequel ledit dispositif de chauffage (8) comprend des sondes de températures (12) attachées à celui-ci.

23. Dispositif selon l'une quelconque des revendications 1 à 22, dans lequel lesdites entrée (5) et sortie (6) ont un tuyau de connexion (5a, 6a) prévu pour le raccordement de tubes.

24. Dispositif selon l'une quelconque des revendications 1 à 23, dans lequel ledit boitier (1) à des brides entourant le haut et la base de la paroi périphérique (3).
